# EUROPEAN PATENT APPLICATION

(11) **EP 0 842 665 A2**
(43) Date of publication of application: **20.05.1998**
(21) Application number: 97309002.0
(22) Date of filing: 10.11.1997
(51) Int. Cl.: A61K 38/48, C12Q 1/37, C12Q 1/68, A61K 38/55, A61K 31/70, A61K 39/395, G01N 33/573

(54) **Interleukin-1 beta converting enzyme like apoptotic proteases and their agonists**

(30) Priority: 14.11.1996 US 31075 P
(71) Applicant: SMITHKLINE BEECHAM CORPORATION, Philadelphia Pennsylvania 19103 (US)
(72) Inventor: Levy, Mark A., SmithKline Beecham Pharma., King of Prussia, Pennsylvania 19406 (US); Nuttall, Mark E., SmithKline Beecham Pharma., King of Prussia, Pennsylvania 19406 (US)
(74) Representative: Crump, Julian Richard John

(57) **Abstract**

This invention relates in part, to use of agonists and antagonists (inhibitors) for the newly identified human interleukin-1 beta converting enzyme like apoptosis proteases (ICE-LAPs).

## Description

### Cross reference to related application

This application claims priority to US provisional application USSN 60/031,075 filed November 14, 1996.

### FIELD OF INVENTION

This invention relates, in part, to newly identified polynucleotides and polypeptides; agonists and antagonists of the polypeptides; and uses of the polynucleotides, polypeptides, agonists and antagonists. In particular, the invention relates to polynucleotides and polypeptides of human interleukin-1 beta converting enzyme like apoptosis proteases hereinafter referred to as "ICE LAPs".

### BACKGROUND OF THE INVENTION

It has recently been discovered that interleukin-1β converting enzyme (ICE) is responsible for cleaving pro-IL-1β into mature and that overexpression of ICE and ICE-like proteins (eg., proteins that are structurally and functionally similar to ICE) can induce programmed cell death (or apoptosis), which is a process through which organisms eliminate unwanted cells.

In the nematode *caenorhabditis elegans,* a genetic pathway of programmed cell death has been identified (Ellis, R.E., et al. Annu. Rev. Cell Biol., 7:663-698 (1991)). Two genes, *ced-3* and *ced-4,* are essential for cells to undergo programmed cell death in C. *elegans* (Ellis, H.M., and Horvitz, H.R., Cell, 44:817-829 (1986)). Recessive mutations that eliminate the function of these two genes prevent normal programmed cell death during the development of C. *elegans.* The function of ced-3 as a novel cysteine protease was first suggested through its sequence homology to ICE. The overall amino acid identity between *ced-3* and ICE is 28%, with a region of 115 amino acids (residues 246-360 of *ced-3* and 164-278 of ICE) that shows the highest identity (43%). This region contains a conserved pentapeptide, QACRG (residues 356-360 of *ced-3),* which contains a cysteine known to be essential for ICE enzymatic activity and biological function.

The similarity between *ced-3* and ICE suggests not only that *ced-3* might function as a cysteine protease but also that ICE might act as a vertebrate programmed cell death gene. *ced-3* and the vertebrate counterpart, ICE, control programmed cell death during embryonic development, (Gagliamini, V. et al., Science, 263:826:828 (1994).

ICE mRNA has been detected in a variety of tissues, including peripheral blood monocytes, peripheral blood lymphocytes, peripheral blood neutrophils, resting and activated peripheral blood T lymphocytes, placenta, the B lymphoblastoid line CB23, and monocytic leukemia cell line THP-1 cells (Cerretti, D.P., et al., Science, 256:97-100 (1992)), suggesting that ICE may have an additional substrates in addition to pro-IL-1β Alternatively, proteins that are related to ICE in function and structure such as the ICE-LAPs, herein described, may be responsible for the molecular events leading to apoptosis. The substrate(s) that these proteases may act upon to cause cell death is presently unknown. One possibility is that it may be a vertebrate homolog of the C. *elegans* cell death gene *ced-4.* Alternatively, one or more of these proteases might directly cause cell death by proteolytically cleaving proteins that are essential for cell viability.

The mammalian gene *bcl-2,* has been found to protect lymphocytes from cell suicide. Also, *crmA,* a cow pox virus gene protein product inhibits ICE's proteinase activity.

Osteoarthritis (OA) is a degenerative disease characterized by progressive erosion of articular cartilage. Chondrocytes are the only cell-type found in articular cartilage and perturbations in metabolism of these cells may be involved in the pathogenesis of OA. Injury to cartilage (chronic or acute) initiates a specific reparative response which involves an increase in the production of proteoglycan and collagen in an attempt to reestablish normal matrix homeostasis. However, with the progress of the disease the 3-dimensional collagen network is disrupted and cell death of chondrocytes occurs in the OA lesions (Malemud and Hering, T.M.: In: Biological Regulation of Chondrocytes. Boca Raton: CRC Press, 1992 295-319). It has been shown that in OA, chondrocytes adjacent to cartilage defects express high levels of bcl-2, a gene involved in inhibition of apoptosis (Erlacher, L. et al., (1995) J. Rheumatology, 22: 926-931). This may represent an attempt to protect chondrocytes from apoptosis induced by the disease process.

Clearly, there is a need for factors that are useful for inducing apoptosis for therapeutic purposes, for example, as an antiviral agent, an anti-tumor agent and to control embryonic development and tissue homeostasis, and the roles of such factors in dysfunction and disease. There also is need for agents that block or prevent apoptosis for therapeutic purposes, for example as an agent in treatment of conditions such as Alzheimer's disease, Parkinson's disease, Huntington's disease, rheumatoid arthritis, osteoarthritis, septic shock, sepsis, stroke, CNS inflammation, osteoporosis, ischemia reperfusion injury, cell death associated with cardiovascular disease, polycystic kidney disease, apoptosis of endothelial cells in cardiovascular disease, degenerative liver disease, MS, ALS, cererbellar degeneration, ischemic injury, myocardial infarction, AIDS, myelodysplastic syndromes, aplastic anemia, male pattern baldness, and head injury damage, as well as aberrant control of embryonic development and tissue homeostasis, follicular lymphomas, carcinomas associated with p53 mutations, autoimmune disorders, such as, for example, SLE, immune-mediated glomerulonephritis; and other cancers, such as, for example, breast cancer, prostate cancer and ovary cancer; and other viral infections, such as, for example, herpesviruses, poxviruses and adenoviruses. There is a need, therefore, for identification and characterization of such factors that are ICE-like proteases, and which can play a role in preventing, ameliorating or correcting dysfunctions or diseases.

The polypeptides of the present invention have conserved residues of interleukin-1 beta converting enzyme apoptosis proteases, and have amino acid sequence homology to known interleukin-1 beta converting enzyme apoptosis proteases. More particularly, the polynucleotides and polypeptides of present invention relate to ICE LAPs -1, 2, 3, 4, 6 and 7, and Mch2 and ICErel-III. When the terminology "ICE LAP" is used hereinafter, it refers to any one of the ICE LAP- 1, 2, 3, 4, 6 or 7, or Mch2 or ICErel-III.

ICE LAPs of the instant invention are described in the following publications or representative GenBank numbers:

ICE-LAP6: "ICE-LAP6, Novel Member of the ICE/Ced-3 Gene Family is Activated byt eh Cytotoxic T cell Protease Granzyme B" H. Guan. et al., (1996) **J. Biological Chemistry** 271: 16720-16724.

ICE-LAP7 (or FLICE): "FLICE: A Novel FADD-Homologous ICE/CED-3-like Protease, is Recruited to the CD95 (Fas/APO-1) Death-Inducing Signaling Complex" M Muzio et al., (1996) **Cell** 85: 817-827.

ICE-LAP 3: "ICE-LAP3, a Novel Mammalian Homologue of the *Caenorhabditis elegans* Cell Death Protein Ced-3 Is Activated during Fas and Tumor Necrosis Factor-induced Apoptosis" H. Duan et al., **J. Biological Chemistry** (1996) 271: 1621-1625; "Mch3, A Novel Human Apoptotic Cysteine Protease Highly Related to CPP32" T. Fernandes-Alnemri, A. Takahashi, R. Armstrong, J. Krebs, L. Fritz, K.J. Tomaselli, L. Wng, Z. Yu, C. M. Croce, G. Salveston, W.C. Eamshaw, G. Litwack, and E.S. Alnemri, (1995) **Cancer Research** 55: 6045-6052

ICE-LAP 4: International application WO 96/13603 published May 9, 1996.

ICE-LAPs 1 and 2: International application WO 96/00297 published January 4, 1996.

ICErel-III: "Molecular Cloning and Pro-apoptotic Activity of ICErel-II and ICErel-III, Members of the ICE/CED-3 Family of Cysteine Proteases" N.A. Munday et al., (1995) **J. Biol. Chem.** 270: 15870-15876. (GenBank number U28015)

Mch2: "Mch2, a New Member of the Apoptotic Ced/Ice Cysteine Protease Gene Family" G. Fernandes-Alnemri et al., (1995) **Cancer Research** 55: 2737-2742. (GenBank number U20536-7)

The following table lists some alternative names used for ICE LAP 1, 2, 3, 4, 6 and 7 and their corresponding GenBank numbers.

| | LAP1 | LAP2 | LAP3 | LAP4 | LAP6 | LAP7 |
|---|---|---|---|---|---|---|
| Alternative names | Ich-2, TX. ICErel II | Ich-1 | Mch-3, CMH-1 | CPP32, Yama | | FLICE, MACH |
| GenBank numbers | U28014 | U13021-2 | U39613 | U13737-8 | U567390 | U58143 |

### SUMMARY OF THE INVENTION

It is an object of the invention to provide the use of ICE LAP polypeptides, particularly human ICE LAP polypeptides, for therapeutic purposes, for example, to treat viral infection, as an anti-tumor agent and to control embryonic development and tissue homeostasis.

In accordance with another object of the invention there are provided products, compositions, processes and methods that utilize the aforementioned polypeptides and polynucleotides for research, biological, clinical, diagnostic and therapeutic purposes, *inter alia.*

In accordance with certain preferred embodiments of this aspect of the invention, there are provided products, compositions and methods, inter alia, for, among other things: assessing ICE LAP expression in cells by determining ICE LAP polypeptides or ICE LAP-encoding mRNA; as an antiviral agent, an anti-tumor agent and to control embryonic development and tissue homeostasis *in vitro, ex vivo* or *in vivo* by exposing cells to ICE LAP polypeptides or polynucleotides as disclosed herein; assaying genetic variation and aberrations, such as defects, in ICE LAP genes; and administering a ICE LAP polypeptide or polynucleotide to an organism to augment ICE LAP function or remediate ICE LAP dysfunction.

In accordance with another aspect of the present invention, there are provided ICE LAP agonists. Among preferred agonists are molecules that mimic ICE LAP, that bind to ICE LAP-binding molecules or receptor molecules, and that elicit or augment ICE LAP-induced responses. Also among preferred agonists are molecules that interact with ICE LAP or ICE LAP polypeptides, or with other modulators of ICE LAP activities and/or gene expression, and thereby potentiate or augment an effect of ICE LAP or more than one effect of ICE LAP. Agonists or inhibitors may be used to treat Alzheimer's disease, Parkinson's disease, Huntington's disease, rheumatoid arthritis, osteoarthritis, septic shock, sepsis, stroke, CNS inflammation. osteoporosis, ischemia reperfusion injury, cell death associated with cardiovascular disease, polycystic kidney disease, apoptosis of endothelial cells in cardiovascular disease, degenerative liver disease, MS, ALS, cererbellar degeneration, ischemic injury, myocardial infarction, AIDS, myelodysplastic syndromes, aplastic anemia, male pattern baldness, and head injury damage, as well as aberrant control of embryonic development and tissue homeostasis, follicular lymphomas, carcinomas associated with p53 mutations, autoimmune disorders, such as, for example, SLE, immune-mediated glomerulonephritis; and other cancers, such as, for example, breast cancer, prostate cancer and ovary cancer; and other viral infections, such as, for example, herpesviruses, poxviruses and adenoviruses.

In a further aspect of the invention there are provided compositions comprising a ICE LAP polynucleotide or a ICE LAP polypeptide for administration to cells *in vitro,* to cells *ex vivo* and to cells *in vivo,* or to a multicellular organism. In certain particularly preferred embodiments of this aspect of the invention, the compositions comprise a ICE LAP polynucleotide for expression of a ICE LAP polypeptide in a host organism for treatment of disease. Particularly preferred in this regard is expression in a human patient for treatment of a dysfunction associated with aberrant endogenous activity of ICE LAP.

Thus the invention relates to a method treating osteoarthritis comprising the step of inhibiting any of the ICE LAPs activity by administering repsective ICE LAP antagonist to a patient in need thereof. One preferred embodiment is a method treating osteoarthritis comprising the step of inhibiting ICE LAP-4 activity by administering ICE LAP-4 antagonist to a patient in need thereof.

### DETAILED DESCRIPTION

Other objects, features, advantages and aspects of the present invention will become apparent to those of skill from the following description. It should be understood. however, that the following description and the specific examples, while indicating preferred embodiments of the invention, are given by way of illustration only. Various changes and modifications within the spirit and scope of the disclosed invention will become readily apparent to those skilled in the art from reading the following description and from reading the other parts of the present disclosure.

For example, the invention also relates methods for utilizing such ICE LAP for the treatment of a susceptibility to Alzheimer's disease, Parkinson's disease, Huntington's disease, rheumatoid arthritis, osteoarthritis, septic shock, sepsis, stroke, CNS inflammation, osteoporosis, ischemia reperfusion injury, cell death associated with cardiovascular disease, polycystic kidney disease, apoptosis of endothelial cells in cardiovascular disease, degenerative liver disease, MS, ALS, cererbellar degeneration, ischemic injury, myocardial infarction, AIDS, myelodysplastic syndromes, aplastic anemia, male pattern baldness, as well as aberrant control of embryonic development and tissue homeostasis, follicular lymphomas, carcinomas associated with p53 mutations, autoimmune disorders, such as, for example, SLE, immune-mediated glomerulonephritis; and other cancers, such as, for example, breast cancer, prostate cancer and ovary cancer; and other viral infections, such as, for example, herpesviruses, poxviruses and adenoviruses. The nucleic acid sequences of ICE LAP may be employed in an assay for ascertaining such susceptibility. Also disclosed are diagnostic assays for detecting diseases related to mutations in the nucleic acid sequences and altered concentrations of the polypeptides. Also disclosed are diagnostic assays for detecting mutations in the polynucleotides encoding the ICE LAP and for detecting altered levels of the polypeptide in a host.

As used herein, "binding molecules" (or otherwise called "interaction molecules") refer to molecules, including receptors, substrates, or ligands, that specifically bind to or interact with polypeptides of the present invention.

### Polynucleotide assays

This invention is also related to the use of ICE LAP polynucleotides to detect complementary polynucleotides for use, for example, as a diagnostic reagent. Detection of a mutated form of ICE LAP associated with a dysfunction will provide a diagnostic tool that can add to or define diagnosis of a disease or susceptibility to a disease which results from under-expression, over-expression or altered expression of ICE LAP. Individuals carrying mutations in the ICE LAP gene may be detected at the DNA level by a variety of techniques. Nucleic acids for diagnosis may be obtained from a patient's cells, such as from blood, urine, saliva, tissue biopsy or autopsy material. The genomic DNA may be used directly for detection or may be amplified enzymatically by using PCR prior to analysis. PCR (Saiki et *al., Nature,* 1986, *324*:163-166), RNA or cDNA may also be used in similar fashion. As an example, PCR primers complementary to the nucleic acid encoding ICE LAP can be used to identify and analyze ICE LAP expression and mutations. For example, deletions and insertions can be detected by a change in size of the amplified product in comparison to the normal genotype. Point mutations can be identified by hybridizing amplified DNA to radiolabeled RNA or, radiolabeled antisense DNA sequences. Perfectly matched sequences can be distinguished from mismatched duplexes by RNase A digestion or by differences in melting temperatures.

Sequence differences between a reference gene and genes having mutations may also be revealed by direct DNA sequencing. In addition, cloned DNA segments may be employed as probes to detect specific DNA segments. The sensitivity of such methods can be greatly enhanced by appropriate use of PCR or other amplification methods. For example, a sequencing primer is used with double-stranded PCR product or a single-stranded template molecule generated by a modified PCR. The sequence determination is performed by conventional procedures with radiolabeled nucleotide or by automatic sequencing procedures with fluorescent-tags.

Genetic testing based on DNA sequence differences may be achieved by detection of alterations in electrophoretic mobility of DNA fragments in gels, with or without denaturing agents. Small sequence deletions and insertions can be visualized by high resolution gel electrophoresis. DNA fragments of different sequences may be distinguished on denaturing formamide gradient gels in which the mobilities of different DNA fragments are retarded in the gel at different positions according to their specific melting or partial melting temperatures (see, e.g., Myers *et al., Science,* 1985, *230*:1242).

Sequence changes at specific locations may also be revealed by nuclease protection assays, such as RNase and S1 protection or the chemical cleavage method (e.g., Cotton *et al., Proc. Natl. Acad. Sci., USA,* 1985, 85: 4397-4401).

Thus, the detection of a specific DNA sequence may be achieved by methods such as hybridization, RNase protection, chemical cleavage, direct DNA sequencing or the use of restriction enzymes, (e.g., restriction fragment length polymorphisms ("RFLP") and Southern blotting of genomic DNA.

In accordance with a further aspect of the invention, there is provided a process for diagnosing or determining a susceptibility to Alzheimer's disease, Parkinson's disease, Huntington's disease, rheumatoid arthritis, osteoarthritis, septic shock, sepsis, stroke, CNS inflammation, osteoporosis, ischemia reperfusion injury, cell death associated with cardiovascular disease, polycystic kidney disease, apoptosis of endothelial cells in cardiovascular disease, degenerative liver disease, MS, ALS, cererbellar degeneration, ischemic injury, myocardial infarction, AIDS, myelodysplastic syndromes, aplastic anemia, male pattern baldness, as well as aberrant control of embryonic development and tissue homeostasis, follicular lymphomas, carcinomas associated with p53 mutations, autoimmune disorders, such as, for example, SLE, immune-mediated glomerulonephritis; and other cancers, such as, for example, breast cancer, prostate cancer and ovary cancer; and other viral infections, such as, for example, herpesviruses, poxviruses and adenoviruses, among others. A mutation in the ICE LAP gene may be indicative of a susceptibility to Alzheimer's disease, Parkinson's disease, Huntington's disease, rheumatoid arthritis, osteoarthritis, septic shock, sepsis, stroke, CNS inflammation, osteoporosis, ischemia reperfusion injury, cell death associated with cardiovascular disease, polycystic kidney disease, apoptosis of endothelial cells in cardiovascular disease, degenerative liver disease, MS, ALS, cererbellar degeneration, ischemic injury, myocardial infarction, AIDS, myelodysplastic syndromes, aplastic anemia, male pattern baldness, as well as aberrant control of embryonic development and tissue homeostasis, follicular lymphomas, carcinomas associated with p53 mutations, autoimmune disorders, such as, for example, SLE, immune-mediated glomerulonephritis; and other cancers, such as, for example, breast cancer, prostate cancer and ovary cancer; and other viral infections, such as, for example, herpesviruses, poxviruses and adenoviruses, among others; and the nucleic acid sequences described above may be employed in an assay for ascertaining such susceptibility. Thus, for example, the assay may be employed to determine a mutation in a ICE LAP gene, as herein described, such as a deletion, truncation, insertion, frame shift, etc., with such mutation being indicative of a susceptibility to a hyperproliferative disease, among others.

The invention provides a process for diagnosing diseases, particularly, Alzheimer's disease, Parkinson's disease, Huntington's disease, rheumatoid arthritis, osteoarthritis, septic shock, sepsis, stroke, CNS inflammation, osteoporosis, ischemia reperfusion injury, cell death associated with cardiovascular disease, polycystic kidney disease, apoptosis of endothelial cells in cardiovascular disease, degenerative liver disease, MS, ALS, cererbellar degeneration, ischemic injury, myocardial infarction, AIDS, myelodysplastic syndromes, aplastic anemia, male pattern baldness, as well as aberrant control of embryonic development and tissue homeostasis, follicular lymphomas, carcinomas associated with p53 mutations, autoimmune disorders, such as, for example, SLE, immune-mediated glomerulonephritis; and other cancers, such as, for example, breast cancer, prostate cancer and ovary cancer; and other viral infections, such as, for example, herpesviruses, poxviruses and adenoviruses, among others; comprising determining from a sample derived from a patient an abnormally decreased or increased level of expression of polynucleotide having the sequence of ICE LAP. Decreased or increased expression of polynucleotide can be measured using any of the methods well known in the art for the quantitation of polynucleotides, such as, for example, PCR, RT-PCR, RNase protection, Northern blotting and other hybridization methods.

In addition to more conventional gel-electrophoresis and DNA sequencing, mutations can also be detected by *in situ* analysis.

### Polypeptide assays

The present invention also relates to a diagnostic assays such as quantitative and diagnostic assays for detecting levels of ICE LAP protein in cells and tissues, including determination of normal and abnormal levels. Thus, for instance, a diagnostic assay in accordance with the invention for detecting over-expression of ICE LAP protein compared to normal control tissue samples may be used to detect the presence of Alzheimer's disease, Parkinson's disease. Huntington's disease, rheumatoid arthritis, osteoarthritis, septic shock, sepsis, stroke, CNS inflammation, osteoporosis, ischemia reperfusion injury, cell death associated with cardiovascular disease, polycystic kidney disease, apoptosis of endothelial cells in cardiovascular disease, degenerative liver disease, MS, ALS, cererbellar degeneration, ischemic injury, myocardial infarction, AIDS, myelodysplastic syndromes, aplastic anemia, male pattern baldness, as well as aberrant control of embryonic development and tissue homeostasis, follicular lymphomas, carcinomas associated with p53 mutations, autoimmune disorders, such as, for example, SLE, immune-mediated glomerulonephritis; and other cancers, such as, for example, breast cancer, prostate cancer and ovary cancer; and other viral infections, such as, for example, herpesviruses, poxviruses and adenoviruses, for example. Assay techniques that can be used to determine levels of a protein, such as an ICE LAP protein of the present invention, in a sample derived from a host are well-known to those of skill in the art. Such assay methods include radioimmunoassays, competitive-binding assays, Western Blot analysis and ELISA assays. Among these ELISAs frequently are preferred. An ELISA assay initially comprises preparing an antibody specific to ICE LAP, preferably a monoclonal antibody. In addition a reporter antibody generally is prepared which binds to the monoclonal antibody. The reporter antibody is attached a detectable reagent such as radioactive, fluorescent or enzymatic reagent, in this example horseradish peroxidase enzyme.

To carry out an ELISA, a sample is removed from a host and incubated on a solid support, e.g. a polystyrene dish. that binds the proteins in the sample. Any free protein binding sites on the dish are then covered by incubating with a non-specific protein such as bovine serum albumin. Next, the monoclonal antibody is incubated in the dish during which time the monoclonal antibodies attach to any ICE LAP proteins attached to the polystyrene dish. Unbound monoclonal antibody is washed out with buffer. The reporter antibody linked to horseradish peroxidase is placed in the dish resulting in binding of the reporter antibody to any monoclonal antibody bound to ICE LAP. Unattached reporter antibody is then washed out. Reagents for peroxidase activity, including a colorimetric substrate are then added to the dish. Immobilized peroxidase, linked to ICE LAP through the primary and secondary antibodies, produces a colored reaction product. The amount of color developed in a given time period indicates the amount of ICE LAP protein present in the sample. Quantitative results typically are obtained by reference to a standard curve.

A competition assay may be employed wherein antibodies specific to ICE LAP attached to a solid support and labeled ICE LAP and a sample derived from the host are passed over the solid support and the amount of label detected attached to the solid support can be correlated to a quantity of ICE LAP in the sample.

### Antibodies

The polypeptides, their fragments or other derivatives, or analogs thereof, or cells expressing them can be used as an immunogen to produce antibodies thereto. These antibodies can be, for example, polyclonal or monoclonal antibodies. The present invention also includes chimeric, single chain, and humanized antibodies, as well as Fab fragments, or the product of an Fab expression library. Various procedures known in the art may be used for the production of such antibodies and fragments.

Antibodies generated against the polypeptides corresponding to a sequence of the present invention can be obtained by direct injection of the polypeptides into an animal or by administering the polypeptides to an animal, preferably a nonhuman. The antibody so obtained will then bind the polypeptides itself. In this manner, even a sequence encoding only a fragment of the polypeptides can be used to generate antibodies binding the whole native polypeptides. Such antibodies can then be used to isolate the polypeptide from tissue expressing that polypeptide.

For preparation of monoclonal antibodies, any technique which provides antibodies produced by continuous cell line cultures can be used. Examples include the hybridoma technique (Kohler, G. and Milstein, C., *Nature* 256. 495-497 (1975), the trioma technique, the human B-cell hybridoma technique (Kozbor et al., *Immunology Today 4:* 72 (1983) and the EBV-hybridoma technique to produce human monoclonal antibodies (Cole et al., pg. 77-96 in *MONOCLONAL ANTIBODIES AND CANCER THERAPY,* Alan R. Liss, Inc. (1985).

Techniques described for the production of single chain antibodies (U.S. Patent No. 4,946,778) can be adapted to produce single chain antibodies to immunogenic polypeptide products of this invention. Also, transgenic mice, or other organisms such as other mammals, may be used to express humanized antibodies to immunogenic polypeptide products of this invention.

The above-described antibodies may be employed to isolate or to identify clones expressing the polypeptide or purify the polypeptide of the present invention by attachment of the antibody to a solid support for isolation and/or purification by affinity chromatography.

Thus, among others, antibodies against ICE LAP may be employed to inhibit the action of such ICE LAP polypeptides, for example, in the treatment of Alzheimer's disease, Parkinson's disease, Huntington's disease, rheumatoid arthritis, osteoarthritis, septic shock, sepsis, stroke, CNS inflammation, osteoporosis, ischemia reperfusion injury, cell death associated with cardiovascular disease, polycystic kidney disease, apoptosis of endothelial cells in cardiovascular disease, degenerative liver disease, MS, ALS, cererbellar degeneration, ischemic injury, myocardial infarction, AIDS, myelodysplastic syndromes, aplastic anemia, male pattern baldness, as well as aberrant control of embryonic development and tissue homeostasis, follicular lymphomas, carcinomas associated with p53 mutations, autoimmune disorders, such as, for example, SLE, immune-mediated glomerulonephritis; and other cancers, such as, for example, breast cancer, prostate cancer and ovary cancer; and other viral infections, such as, for example, herpesviruses, poxviruses and adenoviruses, among others.

### Agonists and antagonists - assays and molecules

The ICE LAPs of the present invention may be employed in a process for screening for compounds which activate (agonists) or inhibit activation and /or protease activity (inhibitors/antagonists) of the polypeptide of the present invention .

For example, a cellular compartment, such as a membrane or a preparation thereof. such as a membrane-preparation, may be prepared from a cell that expresses a molecule that binds ICE LAP, such as a molecule of a signaling or regulatory pathway modulated by ICE LAP. The preparation is incubated with labeled ICE LAP in the absence or presence of a candidate molecule which may be a ICE LAP agonist or antagonist. The ability of the candidate molecule to bind the binding molecule is reflected in a decrease binding of the labeled ligand. Molecules which bind gratuitously, i.e., without inducing the effects of ICE LAP on binding the ICE LAP binding molecule, are most likely good antagonists. Molecules that bind well and elicit effects that are the same as or closely related to ICE LAP are agonists.

ICE LAP-like effects of potential agonists and antagonists may be measured, for instance, by determining activity of a second messenger system following interaction of the candidate molecule with a cell or appropriate cell preparation, comparing the effect with that of ICE LAP or molecules that elicit the same effects as ICE LAP. Second messanger systems that may be useful in this regard include but are not limited to cAMP, guanylate cyclase, ion channel or phosphoinositide hydrolysis second messenger systems.

Another example of an assay for ICE LAP antagonists is a competitive assay that combines ICE LAP and a potential antagonist with membrane-bound ICE LAP binding molecules or recombinant ICE LAP binding molecules under appropriate conditions for a competitive inhibition assay. ICE LAP can be labeled, such as by radioactivity, such that the number of ICE LAP molecules bound to a receptor molecule can be determined accurately to assess the effectiveness of the potential antagonist.

The present invention is further related to a process of screening molecules to identify antagonist/inhibitors or agonists of the ICE-LAPs. Agonists increase the natrural biological function of the protein while antagonists reduce or eliminate such function. An example of such an assay comprises combining an ICE-LAP and a potential antagonists or agonist compound with a cleavable substrate. preferably a protein or peptide, under conditions allowing for action upon the substrate and determining whether the compound prevents cleavage of the substrate or enhances the cleavage. Without limiting the invention in any scope one of many such assays is described in "Identification and inhibition of the ICE/CED3 protease necessary for mammalina apoptosis" (1995) D.W. Nicholson et al., **Nature 376: 37-43.**

Examples of potential ICE LAP antagonists are an antibody, or in some cases an oligonucleotide, which binds to ICE LAP that the activity of the polypeptide is prevented.

Potential antagonists also include proteins which are closely related to the binding molecules (such as substrate) of the ICE LAP, i.e. a fragment of the binding molecules, which have lost biological function and when bind to the ICE LAP polypeptide, inhibit its activity.

A potential antagonist also includes an antisense construct prepared through the use of antisense technology. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes for the mature polypeptides of the present invention, is used to design an antisense RNA oligonucleotide of from about 10 to 40 base pairs in length. A DNA oligonucleotide is designed to be complementary to a region of the gene involved in transcription (triple helix -see Lee et al., *Nucl. Acids Res.,* 6:3073 (1979); Cooney et al, *Science, 241:456* (1988); and Dervan et al., *Science 251:* 1360 (1991)), thereby preventing transcription and the production of ICE LAP polypeptide. The antisense RNA oligonucleotide hybridizes to the mRNA *in vivo* and blocks translation of the mRNA molecule into the ICE LAP polypeptide (antisense - Okano, J. *Neurochem.* 56:560 (1991); Oligodeoxynucleotides as Antisense Inhibitors of Gene Expression, CRC Press, Boca Raton. FL (1988)). The oligonucleotides described above can also be delivered to cells such that the antisense RNA or DNA may be expressed *in vivo* to inhibit production of the ICE LAP polypeptide.

Another potential antagonist is a small molecule which binds to the ICE LAP receptor, making it inaccessible to binding molecules (e.g. substrates) such that normal biological activity is prevented. Examples of small molecules include, but are not limited to, small peptides or peptide-like molecules and small organic molecules.

In general, agonists for ICE LAP polypeptide are employed for therapeutic and prophylactic uses for Alzheimer's disease, Parkinson's disease, Huntington's disease, rheumatoid arthritis, osteoarthritis, septic shock, sepsis, stroke, CNS inflammation, osteoporosis, ischemia reperfusion injury, cell death associated with cardiovascular disease, polycystic kidney disease, apoptosis of endothelial cells in cardiovascular disease, degenerative liver disease, MS, ALS, cererbellar degeneration, ischemic injury, myocardial infarction, AIDS, myelodysplastic syndromes, aplastic anemia, male pattern baldness, as well as aberrant control of embryonic development and tissue homeostasis, follicular lymphomas, carcinomas associated with p53 mutations, autoimmune disorders, such as, for example, SLE, immune-mediated glomerulonephritis; and other cancers, such as, for example, breast cancer, prostate cancer and ovary cancer; and other viral infections, such as, for example, herpesviruses, poxviruses and adenoviruses, among others.

Antagonists/inhibitors for ICE LAP may be employed for a variety of therapeutic and prophylactic uses for Alzheimer's disease, Parkinson's disease, Huntington's disease, rheumatoid arthritis, osteoarthritis, septic shock, sepsis, stroke, CNS inflammation, osteoporosis, ischemia reperfusion injury, cell death associated with cardiovascular disease, polycystic kidney disease, apoptosis of endothelial cells in cardiovascular disease, degenerative liver disease, MS, ALS, cererbellar degeneration, ischemic injury, myocardial infarction, AIDS, myelodysplastic syndromes, aplastic anemia, male pattern baldness, as well as aberrant control of embryonic development and tissue homeostasis, follicular lymphomas, carcinomas associated with p53 mutations, autoimmune disorders, such as, for example, SLE, immune-mediated glomerulonephritis; and other cancers, such as, for example, breast cancer, prostate cancer and ovary cancer; and other viral infections, such as, for example, herpesviruses, poxviruses and adenoviruses.

This invention additionally provides a method of treating an abnormal condition related to an excess of ICE LAP activity which comprises administering to a subject the inhibitor compounds (antagonists) as hereinabove described along with a pharmaceutically acceptable carrier in an amount effective to inhibit its activity by blocking binding of binding molecules ICE LAP polypeptide.

The invention also provides a method of treating abnormal conditions related to an under-expression of ICE LAP activity which comprises administering to a subject a therapeutically effective amount of a compound which activates the polypeptide of the present invention (agonists) as described above in combination with a pharmaceutically acceptable carrier, to thereby alleviate the abnormal conditions.

### Compositions

The invention also relates to compositions comprising the polynucleotide or the polypeptides discussed above or the agonists or antagonists. Thus, the polypeptides of the present invention may be employed in combination with a non-sterile or sterile carrier or carriers for use with cells, tissues or organisms, such as a pharmaceutical carrier suitable for administration to a subject. Such compositions comprise, for instance, a media additive or a therapeutically effective amount of a polypeptide of the invention and a pharmaceutically acceptable carrier or excipient. Such carriers may include, but are not limited to, saline, buffered saline, dextrose, water, glycerol, ethanol and combinations thereof. The formulation should suit the mode of administration.

### Kits

The invention further relates to pharmaceutical packs and kits comprising one or more containers filled with one or more of the ingredients of the aforementioned compositions of the invention. Associated with such container(s) can be a notice in the form prescribed by a governmental agency regulating the manufacture, use or sale of pharmaceuticals or biological products, reflecting approval by the agency of the manufacture, use or sale of the product for human administration.

### Administration

Polypeptides and other compounds of the present invention may be employed alone or in conjunction with other compounds, such as therapeutic compounds.

The pharmaceutical compositions may be administered in any effective, convenient manner including, for instance, administration by topical, oral, anal, vaginal, intravenous, intraperitoneal, intramuscular, subcutaneous, intranasal or intradermal routes among others.

The pharmaceutical compositions generally are administered in an amount effective for treatment or prophylaxis of a specific indication or indications. In general, the compositions are administered in an amount of at least about 10 µg/kg body weight. In most cases they will be administered in an amount not in excess of about 8 mg/kg body weight per day. Preferably, in most cases, dose is from about 10 µg/kg to about 1 mg/kg body weight, daily. It will be appreciated that optimum dosage will be determined by standard methods for each treatment modality and indication, taking into account the indication, its severity, route of administration, complicating conditions and the like.

### Example:

### Aim of experiment:

Test if apoptosis in primary bovine chondrocytes can be induced. Was there involvement of a particular ICE-LAP (caspase) in this process ?

### Methods and Results

Bovine knee joint chondrocytes were isolated and placed in culture. Cells were treated with the topoisomerase inhibitor, camptothecin for 24h. Apoptosis is determined by the cell death ELISA (Boehringer Manheim). There was clear induction both microscopically, by characteristic blebbing, and as a positive readout in the ELISA of apoptosis. Extracts from an experiment run identically, in parallel, were tested for their ability to cleave the peptide substrate DEVD, which is characteristic of a caspase-3 (ICE-LAP-4)-like activity. This activity was undetectable in untreated cells, but after camptothecin treatment was measured at 519 pmol/min/mg of protein.

### Conclusions

Both apoptosis and caspase 3 (ICE-LAP-4)-like activity was induced in primary bovine chondrocytes following treatment of the known apoptotic inducer camptothecin. This suggests a role for caspase 3 (ICE LAP-4) like activity in the apoptosis of chondrocytes, the only cell type found in cartilage. Therefore, in diseases of cartilage, such as osteoarthritis, caspase 3 (ICE LAP-4) is a potential novel therapeutic target.

Thus the invention relates to a method treating osteoarthritis comprising the step of inhibiting any of the ICE LAPs activity by administering repsective ICE LAP antagonist to a patient in need thereof. One preferred embodiment is a method treating osteoarthritis comprising the step of inhibiting ICE LAP-4 activity by administering ICE LAP-4 antagonist to a patient in need thereof.

## Claims

1. A method of treating osteoarthritis comprising the step of administering an ICE LAP-1, 2, 3, 4, 6 or 7 antagonist to a patient in need thereof.

2. A method of claim 2 that is treating osteoarthritis comprising the step of administering ICE LAP-4 antagonist to a patient in need thereof..

3. A method of diagnosing or detecting susceptibility to osteoarthritis; comprising,
a) extracting DNA or RNA from an individual; and
b) detecting the mutation in ICE LAP-1, 2, 3, 4, 6 or 7 nucleotide sequence.

4. The method of claim 3 for which ICE LAP is ICE LAP-4.

5. A method of diagnosing or detecting susceptibility to osteoarthritis comprising,
a) obtaining a tissue sample from an individual;
b) measuring the level of ICE LAP-1, 2, 3, 4, 6 or 7 polypeptide level in the sample; and
c) comparing the measured level of ICE LAP-1, 2, 3, 4, 6 or 7 in the individual with levels measured in control samples to determine whether levels of ICE LAP-1, 2, 3, 4, 6 or 7 are elevated or decreased in the individual.

6. The method of claim 5 for which ICE LAP is ICE LAP-4.
